(19) Europäisches Patentamt
European Patent Office
Office européen des brevets

(11) EP 0 567 847 B1

(12) **EUROPÄISCHE PATENTSCHRIFT**

(45) Veröffentlichungstag und Bekanntmachung des Hinweises auf die Patenterteilung:
**18.09.1996 Patentblatt 1996/38**

(51) Int. Cl.$^6$: **C07C 41/22**, C07B 39/00, C07B 37/04

(21) Anmeldenummer: **93106054.5**

(22) Anmeldetag: **14.04.1993**

# (54) Halogenierungsverfahren in vorteilhaften Lösungsmitteln

Halogenation process in advantageous solvents

Procédé d'halogénation dans des solvants avantageux

(84) Benannte Vertragsstaaten:
**CH DE FR GB IT LI NL**

(30) Priorität: **27.04.1992 DE 4213850**

(43) Veröffentlichungstag der Anmeldung:
**03.11.1993 Patentblatt 1993/44**

(73) Patentinhaber: **BAYER AG**
**51368 Leverkusen (DE)**

(72) Erfinder: **Marhold, Albrecht, Dr.**
**W-5090 Leverkusen 1 (DE)**

(56) Entgegenhaltungen:
**CH-A- 675 122** **DE-A- 2 350 803**
**US-A- 2 516 403**

- **JOURNAL OF ORGANIC CHEMISTRY OF THE USSR. (ZHURNAL ORGANICHESKOI KHIMII) Bd. 26, Nr. 9, September 1986, NEW YORK US Seiten 1261 - 1264 V.A. AVER'YANOV ET AL. 'The Nature of the Selective Action of Aromatic Solvents on Free-Radical Chlorination'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 567 847 B1

Printed by Rank Xerox (UK) Business Services
2.13.4/3.4

## Beschreibung

Die vorliegende Erfindung betrifft Halogenierungsverfahren, die in Gegenwart von vorteilhaften Lösungsmitteln durchgeführt werden.

Halogenierungsreaktionen lassen sich häufig nur in Gegenwart von Lösungsmitteln erfolgreich durchführen. Lösungsmittel für diesen Zweck dürfen unter den angewendeten Bedingungen nicht selbst halogeniert werden und sollten Radikalkettenmechanismen nicht abbrechen, da Halogenierungsreaktionen in vielen Fällen radikalisch ablaufende Reaktionen sind.

Die technisch an Lösungsmittel für Halogenierungsverfahren gestellten Anforderungen werden von Tetrachlorkohlenstoff gut erfüllt, weshalb dieses Lösungsmittel bisher breit angewendet wurde. Jedoch hat Tetrachlorkohlenstoff den Nachteil, daß wegen seiner Toxizität und Flüchtigkeit großer Aufwand nötig ist, um die gesetzlichen Bestimmungen für Arbeitshygiene und Umweltschutz zu erfüllen. Es besteht deshalb ein Bedürfnis nach Lösungsmitteln für Halogenierungen, bei denen weniger oder kein derartiger Aufwand entsteht.

In J. Organic. Chem. of the USSR, 26 (9), Part 2, S. 1261-1264 werden Chlorierungen von Chloralkanen in Gegenwart von Trifluormethylbenzol auf eine Weise beschrieben, die sich für eine Durchführung im industriellen Maßstab nicht eignet.

Es wurde nun ein Verfahren zur Halogenierung von Seitenketten aromatischer und heterocyclischer Verbindungen gefunden, das dadurch gekennzeichnet ist, daß man dabei als Lösungsmittel eine oder mehrere Verbindungen der Formel (I)

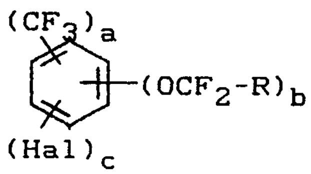

(I),

in der

R für Fluor, Chlor, $CF_3$, $CF_2H$, $CF_2Cl$, $CHFCF_3$, $CF_2CF_3$ oder $CFClCF_3$ steht,

Hal für Fluor oder Chlor steht,

a für Null, 1, 2 oder 3 steht,

b für Null, 1 oder 2 steht und

c für Null oder, im Falle Hal = Fluor auch für 1, 2 oder 3 und im Falle Hal = Chlor auch für 1 steht,

wobei a und b nicht gleichzeitig für Null stehen und die Summe a + b + c höchstens 6 beträgt, einsetzt.

In Formel (I) stehen vorzugsweise

R für Fluor, $CF_3$, $CF_2H$, $CHFCF_3$ oder $CF_2CF_3$,
Hal für Fluor oder Chlor,
a für Null, 1 oder 2,
b für Null oder 1 und
c für Null oder 1,

wobei a und b nicht gleichzeitig für Null stehen.

In Formel (I) stehen besonders bevorzugt

R für Fluor, $CF_2H$ oder $CHFCF_3$,
Hal für Chlor,
a für Null oder 2,
b für Null oder 1 und
c für Null oder 1,

wobei a und b nicht gleichzeitig für Null stehen und die Summe a + b + c höchstens 3 beträgt.

Weiterhin ist es bevorzugt, daß beim Vorhandensein von 2 $CF_3$-Gruppen diese meta-ständig zueinander vorliegen und beim Vorhandensein einer $OCF_2$-R-Gruppe und weiterer Substituenten diese weiteren Substituenten meta- oder para-ständig zur $OCF_2$-R-Gruppe vorliegen.

Die Verbindungen der Formel (I), mit Ausnahme der Verbindungen der Formel (II)

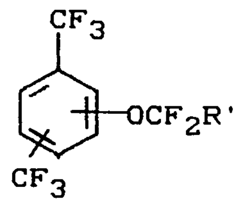

(II),

in der

R' für Fluor, $CF_3$, $CF_2H$, $CHFCF_3$ oder $CF_2CF_3$ steht,

sind im Handel erhältlich oder nach bekannten Verfahren oder analog zu bekannten Verfahren herstellbar (siehe z.B. Pavlat und Leffler, Aromatic Fluorine Compounds, New York (1962), Seite 47 ff. und EP-OS 0 180 818, insbesondere Anspruch 11).

Verbindungen der Formel (II) kann man herstellen, indem man Verbindungen der Formel (III)

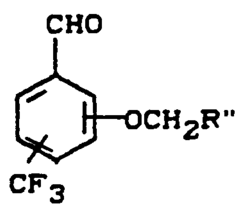

(III),

in der

R" für Wasserstoff, $CF_3$ oder $CHFCF_3$ steht

zunächst mit Phosphorpentachlorid/Chlor chloriert und die Chlorierungsprodukte mit wasserfreiem Fluorwasserstoff oder Antimontrifluorid, gegebenenfalls in Gegenwart eines Katalysators, umsetzt.

Verbindungen der Formel (II) kann man auch herstellen, indem man ein Phenol der Formel (IV)

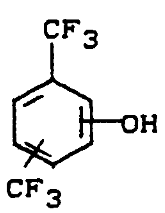

(IV),

mit einem $C_2$- bis $C_3$-Perfluorolefin in Gegenwart einer Base (z.B. wäßriger Natronlauge) umsetzt.

Bei den erfindungsgemäßen Halogenierungen handelt es sich vorzugsweise um Chlorierungen und Bromierungen. Als Halogenierungsmittel kommen z.B. Chlor, Sulfurylchlorid, Brom und N-Bromsuccinimid in Frage.

Die Reaktionsparameter (z.B. Mengenverhältnisse von Halogenierungsmitteln zu Substrat, Temperaturen, Drucke, gegebenenfalls Katalysatoren etc.) können wie bei solchen Reaktionen üblich gewählt werden.

Beispielsweise kann man das erf indungsgemäß einzusetzende Lösungsmittel in Mengen von 5 bis 1 000 Gew.-%, bezogen auf den zu halogenierenden Stoff, einsetzen. Vorzugsweise beträgt diese Menge 20 bis 500 Gew.-%. Man kann beispielsweise den zu halogenierenden Stoff zusammen mit dem erf indungsgemäß zu verwendenden Lösungsmittel vorlegen und anschließend das Halogenierungsmittel zudosieren. Man kann auch das Lösungsmittel, gegebenenfalls zusammen mit einer kleinen Menge des zu halogenierenden Stoffes, vorlegen und den zu halogenierenden Stoff ganz oder zum größten Teil gleichzeitig mit dem Halogenierungsmittel in die Vorlage eindosieren. Man kann den zu halogenierenden Stoff und das Halogenierungsmittel auch auf beliebige andere Weise zusammenführen.

Das Halogenierungsmittel kann beim erf indungsgemäßen Verfahren beispielsweise in der stöchiometrisch erforderlichen Menge oder in einem Überschuß, beispielsweise bis zu 100 Mol-% Überschuß, eingesetzt werden.

Da die erfindungsgemäß einzusetzenden Lösungsmittel höhere Siedepunkte als Tetrachlorkohlenstoff aufweisen, kann bei druckloser Arbeitsweise im Vergleich zu bisher in einem größeren Temperaturbereich gearbeitet werden. Es ist im allgemeinen ausreichend, die erfindungsgemäßen Halogenierungen bei Normaldruck und Temperaturen bis zum Siedepunkt des jeweiligen Lösungsmittels durchzuführen. In besonderen Fällen kann man die Halogenierung bei tieferen oder höheren Temperaturen als dem Siedepunkt des jeweiligen Lösungsmittels (bei Normaldruck) entspricht auch unter Druck durchführen, beispielsweise wenn reaktionsträge Stoffe halogeniert werden sollen und/oder ein verbesserter Stoffübergang des Halogenierungsmittels erreicht werden soll Häufig sind Reaktionstemperaturen im Bereich von 0 bis 180°C anwendbar.

Gegebenenfalls können die bei der jeweiligen Halogenierung üblichen Katalysatoren eingesetzt werden, so z.B. Radikalstarter wie Benzoylperoxid, Di-tert.-butylperoxid und Bis-azo-isobutyronitril.

Die Aufarbeitung der erhaltenen Reaktionsgemische und die Rückgewinnung des erfindungsgemäß eingesetzten Lösungsmittels kann auf an sich bekannte Weise erfolgen, beispielsweise durch Destillation.

Auf die erfindungsgemäße Weise können eine Vielzahl von Halogenierungen vorgenommen werden. Beispielsweise kann man so die Chlorierung oder Bromierung von Alkyl-und Polyalkyl-benzolderivaten in der Seitenkette oder von Heteroaromaten, z.B. Picolin, in der Seitenkette durchführen.

Bevorzugt ist es, auf die erfindungsgemäße Weise die Chlorierung von in Kohlenwasserstoff-, Halogenkohlenwasserstoff-, Alkylether- und/oder Halogenalkylether-Seitenketten enthaltenden Wasserstoffatomen aromatischer Verbindungen durchzuführen.

Das erfindungsgemäße Verfahren hat eine Reihe von überraschenden Vorteilen. Die einzusetzenden Lösungsmittel sind weniger toxisch und weniger flüchtig als Tetrachlorkohlenstoff und erfordern deshalb praktisch keinen besonderen Aufwand auf dem Gebiet der Arbeitshygiene und des Umweltschutzes. Sie können in vielen Fällen wiedergewonnen und praktisch beliebig häufig für Chlorierungen eingesetzt werden. Obwohl die erfindungsgemäß einzusetzenden Lösungsmittel, insbesondere solche der Formel (II), am aromatischen Kern theoretisch einer Halogenierung zugängliche Wasserstoffatome aufweisen, werden unter den üblicherweise beim erfindungsgemäßen Verfahren anzuwendenden Bedingungen keine derartigen Kernchlorierungen beobachtet.

<u>Beispiele</u>

<u>Beispiel 1</u>

In einer Chlorierungsapparatur wurden 50 ml 4-Chlor-trifluormethoxybenzol vorgelegt und 1 g Kaliumchlorid und 1 g Phosphortrichlorid zugegeben. Bei 80°C wurde dann aus einem Tropftrichter eine Lösung von 2 g Azobisisobuttersäurenitril in 120 g 4-Chloranisol zudosiert und gleichzeitig Chlor eingeleitet. Das Ende der Chlorierung wurde gaschromatographisch bestimmt. Bei der anschließenden Destillation wurden 96 Gew.-% des als Lösungsmittel eingesetzten 4-Chlor-trifluormethoxybenzols wiedergewonnen und 186 g 4-Chlor-trichlormethoxybenzol (Siedepunkt 122 bis 126°C bei 16 mbar, Brechungsindex $n_D^{20}$ 1,5565) als Chlorierungsprodukt erhalten, was einer Ausbeute von 89,8 % der Theorie entspricht. Das wiedergewonnene 4-Chlor-trifluormethoxybenzol konnte zusammen mit einer kleinen Zwischenfraktion erneut in diese Chlorierung eingesetzt werden.

<u>Beispiel 2</u>

In 100 ml 1,3-Bistrifluormethylbenzol wurden 0,5 g Kaliumchlorid und 10 g Difluormethoxybenzol vorgelegt und anschließend bei 100°C unter UV-Bestrahlung Chlor eingeleitet. Innerhalb einer Stunde wurden weitere 20 g Difluormethoxybenzol zudosiert. Nach Ende der Chloraufnahme wurde mit Stickstoff ausgeblasen und anschließend destilliert. Es wurden 35,5 g Chlordifluormethoxybenzol isoliert (Siedepunkt 144°C, Brechungsindex $n_D^{20}$ 1,4479).

<u>Beispiel 3</u>

Es wurde wie in Beispiel 2 verfahren, jedoch wurde anstelle des Difluormethoxybenzols 100 g 2,2,2-Trifluorethoxybenzol eingesetzt. Davon wurden 10 g vorgelegt und die restlichen 90 g gemeinsam mit dem Chlor zudosiert. Es wurden 101,5 g 1,1-Dichlor-2,2,2-trifluorethoxybenzol isoliert (Siedepunkt 77 bis 80°C bei 20 mbar, Brechungsindex $n_D^{20}$ 1,4615).

Beispiel 4

In einer Rührapparatur mit Chloreinleitung, Dosiervorrichtung, Intensivkühler und Gasableitung wurden 600 g 1,3-Bistrifluormethylbenzol bei 110°C vorgelegt, anschließend eine Lösung von 18 g Azobisisobuttersäurenitril in 900 g Benzodioxol langsam zudosiert (75 bis 80 g pro Stunde) und gleichzeitig 100 bis 105 g Chlor je Stunde eingeleitet. Die Reaktion war exotherm und wurde durch Entfernung des Heizbades bei 110°C gehalten. Nach vollständiger Eindosierung von Benzodioxol und Chlor wurde noch für weitere 30 Minuten Chlor eingeleitet, anschließend mit Stickstoff ausgeblasen und destilliert. Nach der Abnahme von 630 g einer ersten Fraktion (1,3-Bistrifluormethylbenzol und erneut einsetzbares Chlorierprodukt) wurden 1240 g 2,2-Dichlorbenzodioxol erhalten (Siedepunkt 81 bis 90°C bei 12 mbar).

Beispiel 5

In einer Chlorierungsapparatur wurden 150 g 3,5-Bistrifluormethyl-1,1,2,3,3,3-hexafluorpropoxybenzol vorgelegt und 50 g 4-Methyl-2,2-difluorbenzodioxol zusammen mit 54 g N-Bromsuccinimid und 0,5 g Azobisisobuttersäurenitril für 8 Stunden auf 80 bis 85°C erhitzt. Nach dem Abkühlen wurde der Feststoff abgesaugt und mit wenig Hexan nachgewaschen. Durch Destillation wurde das Lösungsmittel (3,5-Bistrifluormethyl-1,1,2,3,3,3-hexafluorpropoxybenzol) zurückgewonnen und als weitere Fraktion 59,3 g 4-Brommethyl-2,2-difluorbenzodioxol als Bromierungsprodukt erhalten (Siedepunkt 102 bis 104°C bei 20 mbar).

## Patentansprüche

**1.** Verfahren zur Halogenierung von Seitenketten von Alkyl- und Polyalkyl- benzolderivaten und von Seitenketten heteroaromatischer Verbindungen, dadurch gekennzeichnet, daß man dabei als Lösungsmittel eine oder mehrere Verbindungen der Formel (I)

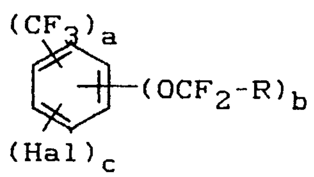

(I),

in der

R für Fluor, Chlor, $CF_3$, $CF_2H$, $CF_2Cl$, $CHFCF_3$, $CF_2CF_3$ oder $CFClCF_3$ steht,

Hal für Fluor oder Chlor steht,

a für Null, 1, 2 oder 3 steht,

b für Null, 1 oder 2 steht und

c für Null oder, im Falle Hal = Fluor auch für 1, 2 oder 3 und im Falle Hal = Chlor auch für 1 steht,

wobei a und b nicht gleichzeitig für Null stehen und die Summe $a + b + c$ höchstens 6 beträgt, einsetzt.

**2.** Verfahren nach Anspruch 1, dadurch gekennzeichnet, daß in Formel (I)

R für Fluor, $CF_3$, $CF_2H$, $CHFCF_3$ oder $CF_2CF_3$,

Hal für Fluor oder Chlor,

a für Null, 1 oder 2,

b für Null oder 1 und

c für Null oder 1 stehen,

wobei a und b nicht gleichzeitig für Null stehen.

**3.** Verfahren nach Ansprüchen 1 und 2, dadurch gekennzeichnet, daß in Formel (I) beim Vorhandensein von zwei $CF_3$-Gruppen diese meta-ständig zueinander vorliegen und beim Vorhandensein einer $OCF_2$-R-Gruppe und weiterer Substituenten diese weiteren Substituenten meta- oder para-ständig zur $OCF_2$-R-Gruppe vorliegen.

**4.** Verfahren nach Ansprüchen 1 bis 3, dadurch gekennzeichnet daß an als Halogenierungsmittel Chlor, Sulfurylchlorid, Brom oder N-Bromsuccinimid einsetzt.

**5.** Verfahren nach Ansprüchen 1 bis 4, dadurch gekennzeichnet, daß man die Verbindungen der Formel (I) in Mengen von 5 bis 1 000 Gew.-%, bezogen auf zu halogenierenden Stoff, einsetzt.

**Claims**

**1.** Process for the halogenation of side chains of alkyl- and polyalkyl-benzene derivatives and side chains of heteroaromatic compounds, characterised in that the solvent used therein comprises one or more compounds of the formula (I)

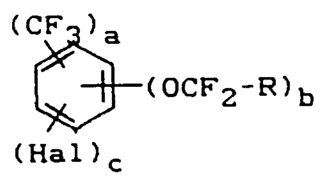

(I)

in which

R represents fluorine, chlorine, $CF_3$, $CF_2H$, $CF_2Cl$, $CHFCF_3$, $CF_2CF_3$ or $CFClCF_3$,

Hal represents fluorine or chlorine,

a represents zero, 1, 2 or 3,

b represents zero, 1 or 2, and

c represents zero or, in the case Hal = fluorine, can also represent 1, 2 or 3, and, in the case Hal = chlorine, can also represent 1,

where a and b do not represent zero simultaneously and the sum $a + b + c$ is at most 6.

**2.** Process according to Claim 1, characterised in that in the formula (I)

R represents fluorine, $CF_3$, $CF_2H$, $CHFCF_3$ or $CF_2CF_3$,

Hal represents fluorine or chlorine,

a represents zero, 1 or 2,

b represents zero or 1, and

c represents zero or 1,

where a and b do not represent zero simultaneously.

**3.** Process according to Claims 1 and 2, characterised in that in the formula (I), in the case of two $CF_3$ groups being present, these are in meta positions with regard to one another and, in the case of an $OCF_2$-R group and further substituents being present, said further substituents are in meta or para positions with regard to the $OCF_2$-R group.

4. Process according to Claims 1 to 3, characterised in that the halogenating agent used is chlorine, sulphuryl chloride, bromine or N-bromosuccinimide.

5. Process according to Claims 1 to 4, characterised in that the compounds of the formula (I) are used in amounts from 5 to 1000% by weight, based on the substance to be halogenated.

**Revendications**

1. Procédé pour halogéner les chaînes latérales de dérivés alkylés et polyalkylés du benzène et les chaînes latérales de composés hétéroaromatiques caractérisé en ce que l'on utilise comme solvant un ou plusieurs composés de formule (I)

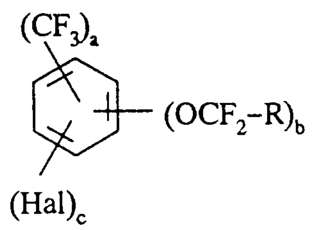

(I),

dans laquelle

R représente le fluor, le chlore, $CF_3$, $CF_2H$, $CF_2Cl$, $CHFCF_3$, $CF_2CF_3$ ou $CFClCF_3$,
Hal représente le fluor ou le chlore,
a représente 0, 1, 2 ou 3,
b représente 0, 1 ou 2 et
c représente 0 ou, dans le cas où Hal = fluor, également 1, 2 ou 3 et, dans le cas où Hal = chlore, également 1,

a et b n'étant pas simultanément nuls et la somme a + b + c étant au plus égale à 6.

2. Procédé selon la revendication 1, caractérisé en ce que, dans la formule (I),

R représente le fluor, $CF_3$, $CF_2H$, $CHFCF_3$ ou $CF_2CF_3$,
Hal représente le fluor ou le chlore,
a représente 0, 1 ou 2,
b représente 0 ou 1 et
c représente 0 ou 1,

a et b n'étant pas simultanément nuls.

3. Procédé selon les revendications 1 et 2, caractérisé en ce que, dans la formule (I), en présence de deux groupes $CF_3$, ceux-ci sont situés en méta l'un par rapport à l'autre et en présence d'un groupe $OCF_2$-R et de substituants supplémentaires, ces substituants supplémentaires sont situés en méta ou en para par rapport au groupe $OCF_2$-R.

4. Procédé selon les revendications 1 à 3, caractérisé en ce que l'on utilise comme agent d'halogénation le chlore, le chlorure de sulfuryle, le brome ou le N-bromosuccinimide.

5. Procédé selon les revendications 1 à 4, caractérisé en ce que l'on utilise les composés de formule (I) en des quantités de 5 à 1 000 % en masse par rapport à la substance à halogéner.